# EUROPEAN PATENT APPLICATION

(11) **EP 4 361 233 A1**
(43) Date of publication of application: **01.05.2024**
(21) Application number: 23205677.0
(22) Date of filing: 25.10.2023
(51) Int. Cl.: C10G 2/00, B01J 38/04, C01B 3/56, C01B 32/40, C10J 3/00, C10J 3/46, C10J 3/72, B01D 53/62

(54) **MANUFACTURING METHOD AND MANUFACTURING APPARATUS OF SYNGAS, AND MANUFACTURING METHOD OF LIQUID HYDROCARBON USING THE SAME**

(30) Priority: 31.10.2022 KR 20220142373
(71) Applicant: SK Innovation Co., Ltd., Seoul 03188 (KR)
(72) Inventor: JEON, Hee Jung, 34124 Daejeon (KR); KIM, Ok Youn, 34124 Daejeon (KR); YUN, Dong Min, 34124 Daejeon (KR)
(74) Representative: Frick, Robert

(57) **Abstract**

The invention relates to a method for manufacturing syngas, the method including: (S1) heat-treating organic wastes under a catalyst in a first reactor to produce a first mixed gas; (S2) separating the catalyst and carbon dioxide (CO₂) from the first mixed gas, and recovering a mixed gas from which the catalyst and the carbon dioxide (CO₂) have been removed; (S3) converting the carbon dioxide (CO₂) separated in (S2) into carbon monoxide (CO) by a reverse Boudouard reaction in a second reactor; and (S4) mixing the mixed gas recovered in (S2) and the carbon monoxide (CO) converted in (S3) to produce syngas.

## Description

### TECHNICAL FIELD

The present invention relates to a manufacturing method and a manufacturing apparatus of syngas, and in particular, to a manufacturing method and a manufacturing apparatus of syngas which may increase a manufacturing yield of syngas and minimize carbon dioxide emission.

### BACKGROUND

Organic wastes may seriously damage the environment by decay when landfilled, and should be discarded through a prescribed treatment process after collecting by their properties when discarded. However, since simple disposal of the organic wastes requires securing treatment facilities and consuming a large amount of manpower and is more wasteful than productive, methods and technologies for recycling organic wastes have been developed in recent years. Representatively, a gasification process technology in which syngas is produced using organic wastes and converted into a high value-added product for energization, may be mentioned.

The gasification process generally refers to a series of processes of reacting carbonaceous raw materials such as coal, organic wastes, and biomass under the supply of water vapor, oxygen, carbon dioxide, or a mixture thereof to convert the raw materials into syngas formed of hydrogen and carbon monoxide as the main components, in which the "syngas" refers to mixed gas which is usually produced by a gasification reaction and contains hydrogen and carbon monoxide as the main component and may further include carbon dioxide and/or methane.

The gasification process technology has expanded to a technology of producing fuels and raw materials of various compounds, and for example, the syngas may be used as the raw material of a Fischer-Tropsch synthesis reaction to manufacture high value-added products such as light oil, heavy oil, diesel oil, jet oil, and lube oil. Besides, it is known that hydrogen in the syngas which is the main product of the gasification process is used to be applied to hydrogen power generation, ammonia manufacture, an oil refining process, and the like, and methanol manufactured from the syngas may be used to obtain high value-added chemicals such as acetic acid, olefin, dimethylether, aldehyde, fuel, and an additive.

Recently, as a process for manufacturing syngas, a gasification process using a catalyst has been carried out, but due to the formation of coke and the like in the gasification process, the catalyst is inactivated to cause process trouble in continuous operation. In addition, for securing economic feasibility, relatively expensive catalysts need to be recovered, but in order to recover catalyst discharged in the state in which coke is agglomerated, a plurality of subsequent processes (such as hot-water extraction and lime digestion) should be carried out, and thus, process efficiency is significantly deteriorated.

Besides, in the gasification reaction of organic wastes, an additional process such as further supplying separate hydrogen may be performed for improving a manufacturing yield, but process efficiency is significantly low, and a separate water-gas shift reaction may be performed, but carbon dioxide as well as hydrogen is produced in a water-gas shift process to cause environmental pollution.

In addition, since the conventionally performed gasification process of organic wastes has a significantly low manufacturing yield of syngas (H₂, CO) which may be converted into a high value-added product and has poor productivity, commercialization using the process is limited. Further, in terms of environmental protection, it is preferred to suppress CO₂ emission, but since the gasification reaction product of organic wastes contains CO₂ in addition to H₂ and CO, carbon dioxide emission is higher than that in landfill or pyrolysis treatment, and thus, the gasification process has a serious problem of rather causing more environmental pollution.

Thus, a manufacturing method and a manufacturing apparatus of syngas which increase a manufacturing yield of syngas which may be converted into a high value-added product and minimize carbon dioxide formation in the gasification process of organic wastes are needed.

### SUMMARY

An aim of the present invention is providing a manufacturing method and a manufacturing apparatus of syngas which have a significantly improved manufacturing yield of syngas from organic wastes.

Another aim of the present invention is providing a manufacturing method and a manufacturing apparatus of syngas which may minimize carbon dioxide formation.

Against this background, the invention relates to a method for manufacturing syngas, wherein the method includes: (S1) heat-treating organic wastes under a catalyst in a first reactor to produce a first mixed gas; (S2) separating the catalyst and carbon dioxide (CO₂) from the first mixed gas, and recovering a second mixed gas from which the catalyst and the carbon dioxide (CO₂) have been removed; (S3) converting the carbon dioxide (CO₂) separated in (S2) into carbon monoxide (CO) by a reverse Boudouard reaction in a second reactor; and (S4) mixing the second mixed gas recovered in (S2) and the carbon monoxide (CO) converted in (S3) to produce syngas.

In an exemplary embodiment, in (S1), when the first mixed gas is produced, a methane reforming reaction in which methane is converted into carbon and hydrogen may occur simultaneously.

In an exemplary embodiment, the first mixed gas produced in (S1) may include hydrogen (H₂), carbon monoxide (CO), and carbon dioxide (CO₂).

In an exemplary embodiment, (S3) may further include (S3-1) introducing the catalyst separated in (S2) to the second reactor and regenerating the catalyst through a reverse Boudouard reaction; and (S3-2) recirculating and resupplying the regenerated catalyst to (S1).

In an exemplary embodiment, in (S1), the catalyst may be a composite catalyst in which at least one active metal selected from nickel, iron, or vanadium is supported on a support.

In an exemplary embodiment, in (S2), the carbon dioxide (CO₂) may be adsorbed on an adsorbent including at least one selected from calcium oxide (CaO), calcium hydroxide (Ca(OH)₂), dolomite, limestone, or trona and separated.

The adsorption of carbon dioxide (CO₂) may be performed at a temperature of 500°C or higher and lower than 800°C and a pressure of 50 to 200 kPa.

In an exemplary embodiment, (S1) may be performed at a temperature of 600 to 900°C and a pressure of 50 to 200 kPa.

In an exemplary embodiment, the reverse Boudouard reaction of (S3) may be performed at a temperature of 800 to 1000°C and a pressure of 50 to 200 kPa.

In an exemplary embodiment, the syngas produced in (S4) may include hydrogen (H₂) and carbon monoxide (CO) and a ratio between the hydrogen (H₂) and the carbon monoxide (CO) may satisfy 1.8 to 2.2.

In an exemplary embodiment, the ratio between the hydrogen (H₂) and the carbon monoxide (CO) may be adjusted by controlling the reverse Boudouard reaction depending on a flow rate of the carbon monoxide (CO) converted in (S3).

In an exemplary embodiment, the ratio between the hydrogen (H₂) and the carbon monoxide (CO) may be adjusted by supplying separate carbon dioxide to (S3) depending on a flow rate of the carbon monoxide (CO) converted in (S3).

In an exemplary embodiment, the organic wastes in (S1) may be at least one selected from waste plastic, solid wastes, biomass, waste oil, or waste tires.

In an exemplary embodiment, before (S2), purifying the first mixed gas produced in (S1) may be further included.

The invention further relates to an apparatus for manufacturing a syngas, wherein the apparatus includes: a first reactor configured to receive organic wastes and allow for a methane reforming reaction and a gasification reaction to simultaneously occur under a catalyst; a carbon dioxide separation unit configured to receive a product from the first reactor and to separate carbon dioxide therefrom; a second reactor configured to receive carbon dioxide separated in the carbon dioxide separation unit and allow for a reverse Boudouard reaction to occur; and a syngas production unit configured to receive and mix a product from which the carbon dioxide has been removed in the carbon dioxide separation unit and carbon monoxide converted in the second reactor to produce syngas.

In an exemplary embodiment, the first reactor may include a fluidized bed reactor.

In an exemplary embodiment, the second reactor may include a fluidized bed reactor.

In an exemplary embodiment, the apparatus may further include a purification unit between the first reactor and a carbon dioxide storage unit.

In an exemplary embodiment, the apparatus may further include a cyclone configured to separate the catalyst from the product of the first reactor between the first reactor and the carbon dioxide separation unit; a supply line configured to supply the catalyst separated from the cyclone to the second reactor; and a recirculation line configured to resupply the regenerated catalyst from the second reactor to the first reactor.

Yet further, the invention relates to a method for manufacturing a liquid hydrocarbon, wherein the method includes: manufacturing a syngas by a method according to the invention; supplying the syngas to a third reactor; and performing a Fischer-Tropsch synthesis reaction in the third reactor.

In an exemplary embodiment, the liquid hydrocarbon may include naphtha having a boiling point of 150°C or lower, Kero having a boiling point of 150 to 265°C, LGO having a boiling point of 265 to 340°C, and VGO having a boiling point of 340°C or higher.

Other features and aspects will be apparent from the following detailed description, the drawings, and the claims.

### BRIEF DESCRIPTION OF THE DRAWINGS

FIG. 1 illustrates a block diagram of a manufacturing method of syngas according to an exemplary embodiment of the present invention including a first reactor, a carbon dioxide separation unit, a second reactor, and a syngas production unit.
FIG. 2 illustrates a block diagram of a manufacturing method of syngas according to an exemplary embodiment of the present invention including a first reactor, a purification unit, a carbon dioxide separation unit, a second reactor, and a syngas production unit.
FIG. 3 illustrates a block diagram of a manufacturing method of syngas according to an exemplary embodiment of the present invention including a first reactor, a cyclone, a purification unit, a carbon dioxide separation unit, a second reactor, and a syngas production unit.
FIG. 4 illustrates a block diagram of a manufacturing method of a liquid hydrocarbon according to an exemplary embodiment of the present invention including a first reactor, a carbon dioxide separation unit, a second reactor, a syngas production unit, and a third reactor.

### [Detailed Description of Main Elements]

1: feed, 100: first reactor, 200: carbon dioxide separation unit, 300: second reactor, 400: syngas production unit, 500: purification unit, 600: cyclone, 700: third reactor

### DETAILED DESCRIPTION OF EMBODIMENTS

The unit of % used in the present specification without particular mention refers to % by weight, unless otherwise defined.

The unit of ppm used without particular mention in the present specification refers to ppm by mass, unless otherwise defined.

A boiling point used without particular mention in the present specification refers to a boiling point at 25°C under 1 atm.

A density used without particular mention in the present specification refers to a density at 25°C under 1 atm.

"Gasification" which is used without particular mention in the present specification refers to a thermal-chemical conversion process through a chemical structure change of a carbonaceous material in the presence of a gasifier (air, oxygen, steam, carbon dioxide, or a mixture thereof) in a broad sense, and refers to a process of converting the carbonaceous material mainly into syngas in a narrower sense.

As a conventional process for manufacturing syngas, a gasification process using a catalyst is carried out, but due to the formation of coke and the like in the gasification process, the catalyst is inactivated to cause process trouble in continuous operation. In addition, for securing economic feasibility, relatively expensive catalysts need to be recovered, but in order to recover catalyst discharged in the state in which coke is agglomerated, a plurality of subsequent processes (such as hot-water extraction and lime digestion) should be carried out, and thus, process efficiency is significantly deteriorated.

Besides, in the case of syngas produced by the gasification reaction, an additional process such as further supplying separate hydrogen may be performed for improving a manufacturing yield, but process efficiency is significantly low, and an additional water-gas shift reaction may be performed, but carbon dioxide as well as hydrogen is produced in the water-gas shift reaction to cause environmental pollution.

In addition, since the conventionally performed gasification process of organic wastes has a significantly low manufacturing yield of syngas which may be converted into a high value-added product of 30% or less and has poor productivity, commercialization using the process is limited. Further, in terms of environmental protection, it is preferred to suppress CO₂ emission, but since the gasification reaction product of organic wastes contains CO₂ in addition to H₂ and CO, carbon dioxide emission is higher than that in landfill or pyrolysis treatment, and thus, the gasification process has a serious problem of rather causing more environmental pollution.

Under consideration of the above, the present invention provides a manufacturing method of syngas including: (S1) heat-treating organic wastes under a catalyst in a first reactor to produce a first mixed gas; (S2) separating the catalyst and carbon dioxide (CO₂) from the first mixed gas, and recovering a second mixed gas from which the catalyst and the carbon dioxide (CO₂) have been removed; (S3) converting the carbon dioxide (CO₂) separated in (S2) into carbon monoxide (CO) by a reverse Boudouard reaction in a second reactor; and (S4) mixing the second mixed gas recovered in (S2) and the carbon monoxide (CO) converted in (S3) to produce syngas. Formation of carbon dioxide is minimized by the manufacturing method as compared with the conventional method, thereby preventing environmental pollution and significantly improving a manufacturing yield of syngas from organic wastes.

In (S1), the organic wastes are heat treated under a catalyst in a first reactor to produce a first mixed gas, and usually, at least one reaction selected from the following Reaction Formulae 1 to 4 is involved to produce the first mixed gas.

[Reaction Formula 1] C + H₂O → H₂ + CO (water gasification reaction of carbon)

[Reaction Formula 2] C + CO₂ → 2CO (carbon dioxide gasification reaction of carbon)

[Reaction Formula 3] CO + 3H₂ → CH₄ + H₂O (methanation reaction)

[Reaction Formula 4] C + O₂ → CO₂ (oxidation reaction of carbon).

In an exemplary embodiment, in (S1), when the first mixed gas is produced, a methane reforming reaction in which methane is converted into carbon and hydrogen may occur simultaneously. The first mixed gas produced in (S1) may include hydrogen (H₂), carbon monoxide (CO), and carbon dioxide (CO₂). Specifically, the product of (S1) may include H₂, CO, CH₄, and CO₂ including moisture, and when the first mixed gas is produced, the methane reforming reaction occurs simultaneously, and thus, production yields of H₂ and CO which are the main components of syngas which may be converted into high value-added products may be significantly improved. The methane reforming reaction may involve at least one selected from the following Reaction Formulae 5 to 7:

[Reaction Formula 5] CH₄ → C + 2H₂ (methane reforming reaction)

[Reaction Formula 6] CH₄ + H₂O → CO + 3H₂ (steam modification reaction of methane)

[Reaction Formula 7] CH₄ + CO₂ → 2CO + 2H₂ (carbon dioxide reforming reaction of methane).

It is favorable that (S1) is performed under a water vapor atmosphere by supplying the water vapor atmosphere to the first reactor. For improving reaction efficiency, a small amount of carbon dioxide may be also optionally supplied in addition to the water vapor, and the water vapor amount in the first reactor may be adjusted by adjusting partial pressure using oxygen and, if required, inert gas such as nitrogen and argon. As described later, (S1) may be performed at a temperature of 600 to 900°C and a pressure of 50 to 200 kPa. Under the conditions, gasification efficiency and methane reformation efficiency may be excellent. Specifically, the temperature may be 700 to 900°C and the pressure may be 100 to 200 kPa, and more specifically, the temperature may be 850 to 900°C and the pressure may be 150 to 200 kPa.

That is, since the methane reforming reaction of Reaction Formulae 5 to 7 occurs simultaneously with the gasification reaction of Reaction Formulae 1 to 4 in (S1), it is very favorable in terms of economic efficiency and energy efficiency while solving the problem of a significantly low manufacturing yield of syngas during the conventional gasification process of organic wastes. In addition, since the syngas is manufactured by a series of processes including (S1) to (S4) described later, carbon dioxide emission is minimized to prevent environmental pollution and also the manufacturing yield of syngas may be maximized.

The first reactor may include a fluidized bed reactor. It is preferred to use the fluidized bed reactor in terms of gasification efficiency and a catalyst regeneration process described later. In the fluidized bed reactor, a solid bed (bed material) is fluidized and mixed in a suspended state due to reaction gas having an upward flow to cause a gasification reaction, and specifically, the first reactor may include a fluidized bed reactor, in which the fluidized bed reactor may be a riser.

Through the process of (S2), in order to recover syngas from the product of (S1), the catalyst and the carbon dioxide (CO₂) are separated, and the second mixed gas from which the catalyst and the carbon dioxide (CO₂) have been removed may be recovered. In the separation of the catalyst and carbon dioxide (CO₂), it is preferred to separate the catalyst first and then separate carbon dioxide (CO₂), and the catalyst and carbon dioxide (CO₂) may be separated at the same time. Preferably, as described later, it is preferred that a cyclone is provided between the first reactor and a carbon dioxide separation unit to separate the catalyst from the product of the first reactor (first mixed gas), and then the product from which the catalyst has been separated is introduced to the carbon dioxide separation unit to separate carbon dioxide and recover the second mixed gas.

Through the process of (S3), the carbon dioxide (CO₂) separated in (S2) may be converted into carbon monoxide (CO) by a reverse Boudouard reaction in the second reactor. The carbon dioxide produced by the heat treatment process of (S1) is not discharged as it is, but is converted into carbon monoxide, thereby preventing environmental pollution and also improving the manufacturing yield of syngas. The reverse Boudouard reaction may involve the reaction of the following Reaction Formula 8. In order to perform the reverse Boudouard reaction well, carbon such as activated carbon may be supplied, or as described later, in (S1), a catalyst inactivated by coke and the like is used to perform the reverse Boudouard reaction.

[Reaction Formula 8] C + CO₂ → 2CO (reverse Boudouard reaction)

The carbon monoxide which has been converted by the reverse Boudouard reaction of (S3) may be used in the production of syngas in (S4) described later. When unreacted carbon dioxide is present in the product of (S3), carbon dioxide is separated again from the product, and the reverse Boudouard reaction of (S3) may be repeated once or more on the separated carbon dioxide. Accordingly, carbon dioxide may be converted into carbon monoxide in a very high yield.

Through the process of (S4), the second mixed gas recovered in (S2) and the carbon monoxide (CO) converted in (S3) may be mixed to produce syngas. As described above, the syngas contains CO and H₂ as main components, and the syngas is used as a raw material of a Fischer-Tropsch reaction to manufacture high value-added products, in which the stoichiometrically required ratio of H₂:CO ratio is preferably about 2:1.

That is, in the present invention, since syngas is manufactured by a series of processes including (S1) to (S4), the manufacturing yield of syngas is significantly increased as compared with the conventional gasification process of the organic wastes and also carbon dioxide emission is minimized to prevent environmental pollution.

In an exemplary embodiment, (S3) may further include (S3-1) introducing the catalyst separated in (S2) to the second reactor and regenerating the catalyst through a reverse Boudouard reaction; and (S3-2) recirculating and resupplying the regenerated catalyst to (S1). As described above, the catalyst is inactivated by occurrence of coke and the like which are by-products in the conventional gasification process, so that process trouble occurs by the inactivated catalyst during continuous operation. In order to solve the problem, a certain amount of a new catalyst is continuously exchanged with a waste catalyst to maintain a catalytic activity at a certain level or higher, but process efficiency is significantly deteriorated in the exchange process. In the present invention, carbon dioxide is converted into carbon monoxide in the reverse Boudouard reaction, and simultaneously a waste catalyst in which coke and the like are agglomerated is treated with the carbon dioxide to regenerate the catalyst, which is recirculated to (S1), resupplied, and used, thereby converting carbon dioxide emitted to the outside into carbon monoxide to improve the manufacturing yield of syngas and also promoting the regeneration of the waste catalyst to significantly improve process efficiency. Herein, the second reactor may include the fluidized bed reactor, and for example, a regenerator in the fluidized bed reactor.

In an exemplary embodiment, in (S1), the catalyst may be a composite catalyst in which at least one active metal selected from nickel, iron, or vanadium is supported on a support. The support may be any support having durability to support active metal, and for example, may be selected from silicon, aluminum, zirconium, sodium, manganese, titanium, oxides thereof, and the like. Specifically, in terms of gasification and methane reforming efficiency, the active metal may be nickel, and the support may be silicon dioxide.

In an exemplary embodiment, in (S2), the carbon dioxide (CO₂) may be adsorbed on an adsorbent including at least one selected from calcium oxide (CaO), calcium hydroxide (Ca(OH)₂), dolomite, limestone, or trona and separated. Carbon dioxide may be effectively separated from the first mixed gas by the adsorbent. Specifically, the adsorbent may be calcium oxide.

The adsorption of carbon dioxide (CO₂) may be performed at a temperature of 500°C or higher and lower than 800°C and a pressure of 50 to 200 kPa. Under the conditions, carbon dioxide adsorption efficiency may be excellent. Specifically, the temperature may be 500 to 700°C and the pressure may be 50 to 150 kPa, and more specifically, the temperature may be 550 to 650°C and the pressure may be 50 to 100 kPa.

The carbon dioxide adsorbed on the adsorbent is desorbed again and may be used as a reactant of the reverse Boudouard reaction of (S3). The desorption may be performed at a temperature of higher than 500 and 1000°C or less and a pressure of 50 to 200 kPa. Under the conditions, carbon dioxide desorption efficiency may be excellent. Specifically, the temperature may be 700 to 950°C and the pressure may be 50 to 150 kPa, and more specifically, the temperature may be 850 to 950°C and the pressure may be 50 to 100 kPa.

In an exemplary embodiment, (S1) may be performed at a temperature of 600 to 900°C and a pressure of 50 to 200 kPa. Under the conditions, gasification efficiency and methane reformation efficiency may be excellent. Specifically, the temperature may be 700 to 900°C and the pressure may be 100 to 200 kPa, and more specifically, the temperature may be 850 to 900°C and the pressure may be 150 to 200 kPa.

In an exemplary embodiment, the reverse Boudouard reaction of (S3) may be performed at a temperature of 800 to 1000°C and a pressure of 50 to 200 kPa. Under the conditions, the conversion efficiency from carbon dioxide into carbon monoxide and the catalyst regeneration efficiency may be excellent. Specifically, the temperature may be 800 to 1000°C and the pressure may be 50 to 150 kPa, and more specifically, the temperature may be 950 to 1000°C and the pressure may be 100 to 150 kPa.

In an exemplary embodiment, the syngas produced in (S4) may include hydrogen (H₂) and carbon monoxide (CO) and a ratio between the hydrogen (H₂) and the carbon monoxide (CO) may satisfy 1.8 to 2.2. As described above, the syngas is used as a raw material of the Fischer-Tropsch reaction and converted into high value-added products such as light oil, heavy oil, diesel oil, jet oil, and lube oil, and when a stoichiometrically required ratio of H₂:CO satisfies 1.8 to 2.2, conversion efficiency may be excellent. Preferably, the ratio of H₂:CO may be 1.9 to 2.1, more preferably 1.95 to 2.05.

In an exemplary embodiment, the ratio between the hydrogen (H₂) and the carbon monoxide (CO) may be adjusted by controlling the reverse Boudouard reaction depending on a flow rate of the carbon monoxide (CO) converted in (S3). A flow rate of the carbon monoxide converted in (S3) is measured in real time and the reverse Boudouard reaction is controlled so that the ratio of H₂:CO is satisfied. The reaction conditions and the reaction rate of the reverse Boudouard reaction in the second reactor are controlled, thereby satisfying the H₂:CO ratio to improve process efficiency. In addition, when the flow rates of H₂ and CO in the second mixed gas recovered in (S2) are measured together, process efficiency may be more improved.

In an exemplary embodiment, the ratio between the hydrogen (H₂) and the carbon monoxide (CO) may be adjusted by controlling the reverse Boudouard reaction by supplying separate carbon dioxide to (S3) depending on a flow rate of the carbon monoxide (CO) converted in (S3). A flow rate of the carbon monoxide converted in (S3) is measured in real time so that the ratio of H₂:CO is satisfied and separate carbon dioxide may be supplied and adjusted. Specifically, when CO is significantly small in the ratio of H₂:CO, carbon dioxide is separately supplied to (S3), thereby improving the reverse Boudouard reaction activity to satisfy the ratio of H₂:CO. The supply of carbon dioxide may be performed by a separate carbon dioxide storage unit.

In an exemplary embodiment, the organic wastes in (S1) may be at least one selected from waste plastic, solid wastes, biomass, waste oil, or waste tires. Any carbon-containing material such as biomass and coal may be used as the raw material of a gasification reaction in principle, but considering the problem to be solved in the present invention, it may be appropriate to use the organic wastes.

In an exemplary embodiment, before (S2), purifying the first mixed gas produced in (S1) may be further included. The first mixed gas produced in (S1) may further include aqueous impurity gas components such as H₂S, HCl, HOCl, and NH₃, in addition to H₂, carbon dioxide (CO), and carbon dioxide (CO₂). Thus, impurity gas components are removed by the process of purifying the first mixed gas, a manufacturing yield of syngas may be further improved. Besides, before (S2), when H₂ is relatively insufficiently produced in the gasification process depending on the kind of organic wastes, a water gas shift (WGS) reaction is performed to replenish H₂, thereby improving the manufacturing yield of syngas. The WGS reaction may be performed in the conditions of 20 to 70 bar and 100 to 250°C under a catalyst. The catalyst may be used without limitation as long as it is a catalyst having WGS reaction activity, and preferably, may be a Cu-Zn mixed catalyst. Herein, since carbon dioxide produced by the water gas shift reaction is converted into carbon monoxide by the reverse Boudouard reaction, the environmental pollution problem caused by performing the conventional water gas shift reaction may be solved and simultaneously the effect of improving a manufacturing yield of syngas may be promoted.

In addition, the present invention provides a manufacturing apparatus of syngas including: a first reactor 100 where organic wastes are introduced and a methane reforming reaction and a gasification reaction are simultaneously performed under a catalyst; a carbon dioxide separation unit 200 where a product is introduced from the first reactor 100 and carbon dioxide is separated; a second reactor 300 where the carbon dioxide separated from the carbon dioxide separation unit 200 is introduced and a reverse Boudouard reaction is performed; and a syngas production unit 400 where a product from which the carbon dioxide has been removed in the carbon dioxide separation unit 200 and carbon monoxide converted in the second reactor 300 are mixed to produce syngas. The syngas may be manufactured in a high yield from the organic wastes by the apparatus, and carbon dioxide emission is minimized to prevent environmental pollution.

In an exemplary embodiment, the first reactor 100 may include a fluidized bed.

In an exemplary embodiment, the second reactor 300 may include a fluidized bed.

In the fluidized bed reactor, a solid bed (bed material) is fluidized and mixed in a suspended state due to reaction gas having an upward flow to cause a gasification reaction, and specifically, the first reactor 100 may be a riser in the fluidized bed reactor. Organic wastes are supplied into the first reactor 100, and may be pyrolyzed under a catalyst provided in the reactor or pyrolyzed under a catalyst which is supplied through a resupply line connected from the second reactor 300 to the first reactor 100 and regenerated. Herein, the second reactor 300 may be a regenerator in the fluidized bed reactor.

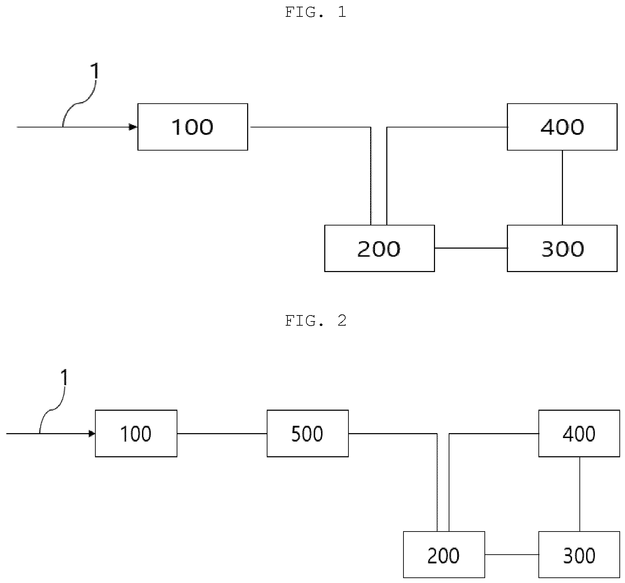

As shown in FIG. 1, an organic waste 1 is introduced into the first reactor and heat-treated under a catalyst to perform the gasification reaction and the methane reforming reaction simultaneously. The product (first mixed gas) produced in the first reactor 100 may include hydrogen (H₂), carbon monoxide (CO), and carbon dioxide (CO₂). The product is introduced from the first reactor 100 to the carbon dioxide separation unit 200 to separate carbon dioxide from the product.

The separated carbon dioxide may be introduced from the carbon dioxide separation unit 200 to the second reactor 300 and converted into carbon monoxide by the reverse Boudouard reaction.

The product from which carbon dioxide has been removed in the carbon dioxide separation unit 200 and the carbon monoxide converted in the second reactor 300 may be mixed in the syngas production unit 400 to produce the syngas. Herein, when unreacted carbon dioxide remains in the product of the second reactor 300, the product is introduced again to the carbon dioxide separation unit 200 to separate carbon dioxide from the product, and the separated carbon dioxide may be introduced again to the second reactor 300 to perform the reverse Boudouard reaction again in the second reactor 300. The process may be repeated once or more, thereby converting unreacted carbon dioxide into carbon monoxide to increase a carbon monoxide conversion rate.

In an exemplary embodiment, a purification unit 500 may be further included between the fluidized bed reactor and the carbon dioxide storage unit, as shown in FIG. 2. In the purification unit 500, the purification may be performed by a dust collection filter for high temperature/high pressure or a wet scrubber, and aqueous impurity gas components such as H₂S, HCl, HOCl, and NH₃ are removed to further improve the manufacturing yield of syngas.

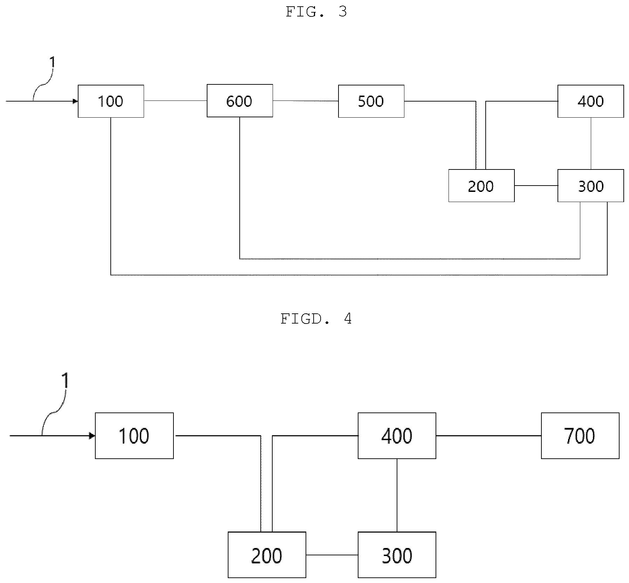

In an exemplary embodiment, the manufacturing apparatus of syngas may further include a cyclone 600 which separates the catalyst from the product of the first reactor 100; a supply line which supplies the catalyst separated from the cyclone 600 to the second reactor 300; and a recirculation line which resupplies the regenerated catalyst from the second reactor 300 to the first reactor 100, as shown in FIG. 3. The catalyst separated from the cyclone 600 is introduced to the second reactor 300, in which the catalyst may include a coke component produced in the pyrolysis process of the organic wastes. Meanwhile, carbon dioxide may be introduced from the carbon dioxide separation unit 200 to the second reactor 300. Besides, the separate carbon dioxide storage unit may be provided to introduce separate carbon dioxide. The catalyst is treated with carbon dioxide at a high temperature of 800°C or higher, thereby removing impurities such as coke included in the catalyst and regenerating the catalyst. The regenerated catalyst may be reintroduced to the first reactor 100 through the recirculation line.

In addition, the present invention provides a manufacturing method of a liquid hydrocarbon including: supplying syngas to a third reactor 700; and performing a Fischer-Tropsch synthesis reaction in the third reactor 700, wherein the syngas is the syngas produced in claim 1. As shown in FIG. 4, in the third reactor 700, the syngas may be supplied from the syngas production unit 400, and the syngas produced through (S1) to (S4) may be used as a raw material of the Fischer Tropsch reaction represented by the following Reaction Formula 9, thereby manufacturing a liquid hydrocarbon.

[Reaction Formula 9] nCO + 2nH₂ → CₙH₂ₙ + nH₂O

The third reactor 700 may be a Fischer-Tropsch synthesis reactor, in which the third reactor 700 may include a liquid product separation and a heavy end recovery unit. Besides, a fixed bed reactor or a micro channel reactor may be applied to the third reactor 700. In order to improve a manufacturing yield of the liquid hydrocarbon, it is preferred that a stoichiometric ratio of H₂:CO satisfies 1.8 to 2.2. Preferably, the ratio of H₂:CO may be 1.9 to 2.1, more preferably 1.95 to 2.05. The manufactured liquid hydrocarbon may include naphtha having a boiling point of 150°C or lower, Kero having a boiling point of 150 to 265°C, LGO having a boiling point of 265 to 340°C, and VGO having a boiling point of 340°C or higher. The syngas produced in the conventional gasification process of organic wastes has a low amount of syngas (H₂, CO) which may be converted into a product (liquid hydrocarbon) and a lower molar mass of H₂ compared to CO in H₂:CO, and thus, a recovery rate of the expected product is significantly deteriorated. The syngas produced by the steps of (S1) to (S4) of the present invention is used as the raw material of the Fischer-Tropsch synthesis reaction, thereby improving the recovery rate of the liquid hydrocarbon product as compared with the conventional gasification process of organic wastes.

For the matters which are not described in the manufacturing apparatus of syngas, the above description of the manufacturing method of syngas may be referred.

Hereinafter, the present invention will be described in detail by the examples.

### Example 1

A syngas manufacturing apparatus including a first reactor, a purification unit, a carbon dioxide separation unit, a second reactor, and a syngas production unit was operated for 120 minutes to manufacture syngas. Specifically, 1000 g of municipal solid wastes was added to the first reactor (fluidized bed reactor) equipped with a Ni/SiO₂ catalyst, and then heat-treated at a temperature of 800°C and a pressure of 100 kPa under water vapor conditions. The product of the first reactor (first mixed gas) was separated from the catalyst, and the product separated from the catalyst was added to the purification unit (wet scrubber) and treated at 70°C and 100 kPa to remove impurities. Thereafter, the product was added to a carbon dioxide separation unit equipped with CaO to separate carbon dioxide. Specifically, the carbon dioxide was adsorbed to CaO at 650°C and 100 kPa and separated, and the product from which the carbon dioxide has been removed (second mixed gas) was separately recovered. The CaO was treated again under the conditions of 850°C and 100 kPa to desorb the carbon dioxide. The desorbed carbon dioxide was added to the second reactor (regenerator) and a reverse Boudouard reaction was carried out under conditions of 900°C and 100 kPa to convert the carbon dioxide into carbon monoxide.

The second mixed gas from which the carbon dioxide has been removed and the carbon monoxide converted in the second reactor were introduced to a syngas production unit and mixed at 200°C to manufacture syngas. In this process, a flow rate of the carbon monoxide converted in the second reactor was measured in real time, the activity of the reverse Boudouard reaction was adjusted depending on the amount of the converted carbon monoxide, and synthesis was performed so that a ratio of H₂:CO was 2 when syngas was produced.

In addition, the separated catalyst was supplied to the second reactor (regenerator) through a separate supply line, treated with the reverse Boudouard reaction, regenerated, and then resupplied to the first reactor through a recirculation line connected to the first reactor.

### Example 2

Syngas was manufactured in the same manner as in Example 1, except that the reaction was performed at a temperature of 850°C and pressure of 150 kPa in the first reactor and a temperature of 950°C and a pressure of 150 kPa of in the second reactor.

### Example 3

Syngas was manufactured in the same manner as in Example 1, except that when the syngas was manufactured, the reaction was performed without measuring the flow rate of the carbon monoxide converted in the second reactor in real time.

### Example 4

Syngas was manufactured in the same manner as in Example 1, except that the reaction was performed without the recirculation of the catalyst without performing the regeneration process of the separated catalyst.

### Comparative Example 1

Syngas was manufactured in the same manner as in Example 1, except that the heat treatment process of the first reactor was performed without the catalyst.

### Comparative Example 2

Syngas was manufactured in the same manner as in Example 1, except that the reaction was performed without performing the reverse Boudouard reaction using the manufacturing apparatus of syngas including no second reactor.

### Evaluation Example

Gas chromatography (GC) was performed on the syngas manufactured by operating manufacturing apparatus of syngas for 120 minutes to analyze the composition. Specifically, the syngas was quantified by GC to calculate selectivity for each gas, and the composition of each gas was analyzed by the total amount of gas confirmed from a flowmeter, thereby evaluating the gas manufacturing yield and a carbon dioxide reduction effect.

The evaluation results are shown in the following Table 1:

**[Table 1]**

| | | Example 1 | Example 2 | Example 3 | Example 4 | Comparative Example 1 | Comparative Example 2 |
|---|---|---|---|---|---|---|---|
| H₂ | Molar rate (lbmol/hr) | 1006 | 1128 | 1001 | 930 | 725 | 925 |
| | Composition (vol%) | 45 | 48 | 45 | 44 | 36 | 43 |
| CO | Molar rate (lbmol/hr) | 503 | 574 | 478 | 459 | 412 | 210 |
| | Composition (vol%) | 23 | 24 | 22 | 21 | 21 | 10 |
| CO₂ | Molar rate (lbmol/hr) | 609 | 548 | 624 | 635 | 656 | 884 |
| | Composition (vol%) | 27 | 24 | 28 | 30 | 32 | 41 |
| Others | Molar rate (lbmol/hr) | 110 | 95 | 107 | 112 | 197 | 135 |
| | Composition (vol%) | 5 | 4 | 5 | 5 | 11 | 6 |

Referring to the results of analyzing the syngas composition of Table 1, it was confirmed that in the manufacturing process of syngas, Examples 1 to 4 had much better production amounts of H₂ and CO, and had reduced production amounts of CO₂ to have a favorable effect in terms of a syngas manufacturing yield and environmental pollution prevention, as compared with Comparative Examples 1 and 2.

Specifically, it was confirmed in Example 2 that as the temperature conditions were changed, H₂ and CO production amounts were best with H₂ being 1128 lbmol/hr and CO being 574 lbmol/hr, and the ratio of H₂:CO satisfied about 2:1.

It was confirmed in Example 3 that since the reaction was performed without measuring the flow rate of carbon monoxide converted in the second reactor in real time, the ratio of H₂:CO did not satisfy 2:1 and the production amounts of H₂ and CO were somewhat lower than those of Example 1 with H₂ being 1101 lbmol/hr and CO being 478 lbmol/hr, but were higher than those of Comparative Examples 1 and 2.

Since in Example 4, the reaction was performed without performing the regeneration process of the separated catalyst and without recirculation of the catalyst, it was confirmed that the production amount of H₂ and CO were somewhat lower than those of Example 1 with H₂ being 930 lbmol/hr and CO being 459 lbmol/hr as compared with those of Example 1, but were higher than those of Comparative Examples 1 and 2.

Since in Comparative Example 1, the heat treatment process was performed without the catalyst to decrease the simultaneous reaction efficiency of gasification and methane reformation, it was confirmed that the production amounts of H₂ and CO were much lower than those of Example 1 with H₂ being 725 lbmol/hr and CO being 412 lbmol/h.

Since in Comparative Example 2, the reaction was performed without performing the reverse Boudouard reaction by using the manufacturing apparatus of syngas including no second reactor, it was confirmed that considering that the CO production amount was much lower and the CO₂ production amount was much higher than those of Example 1 with H₂ being 925 lbmol/hr, CO being 210 lbmol/hr, and CO₂ being 884 lbmol/hr, and thus, it is not preferred in terms of a manufacturing yield of syngas and environmental pollution.

The manufacturing method and apparatus of syngas according to the present invention may have a significantly improved manufacturing yield of syngas from organic wastes. They may minimize carbon dioxide formation in a syngas manufacturing process. They may also induce an effect of regenerating a waste catalyst in a process of converting carbon dioxide into carbon monoxide to improve process efficiency.

## Claims

1. A method for manufacturing syngas, the method comprising:
(S1) heat-treating organic wastes under a catalyst in a first reactor (100) to produce a first mixed gas;
(S2) separating the catalyst and carbon dioxide (CO₂) from the first mixed gas, and recovering a second mixed gas from which the catalyst and the carbon dioxide (CO₂) have been removed;
(S3) converting the carbon dioxide (CO₂) separated in (S2) into carbon monoxide (CO) by a reverse Boudouard reaction in a second reactor (300); and
(S4) mixing the second mixed gas recovered in (S2) and the carbon monoxide (CO) converted in (S3) to produce syngas.

2. The method of claim 1, wherein the first mixed gas produced in (S1) includes hydrogen (H₂), carbon monoxide (CO), and carbon dioxide (CO₂).

3. The method of any preceding claim, wherein
(S3) further includes:
(S3-1) introducing the catalyst separated in (S2) to the second reactor (300) and regenerating the catalyst through a reverse Boudouard reaction; and
(S3-2) recirculating and resupplying the regenerated catalyst to (S1).

4. The method of any preceding claim, wherein in (S1), the catalyst is a composite catalyst in which at least one active metal selected from nickel, iron, or vanadium is supported on a support.

5. The method of any preceding claim, wherein in (S2), the carbon dioxide (CO₂) is adsorbed on an adsorbent including at least one selected from calcium oxide (CaO), calcium hydroxide (Ca(OH)₂), dolomite, limestone, or trona and separated, and wherein the adsorption of carbon dioxide (CO₂) is preferably performed at a temperature of 500°C or higher and lower than 800°C and a pressure of 50 to 200 kPa.

6. The method of any preceding claim, wherein (S1) is performed at a temperature of 600 to 900°C and a pressure of 50 to 200 kPa.

7. The method of any preceding claim, wherein the reverse Boudouard reaction of (S3) is performed at a temperature of 800 to 1000°C and a pressure of 50 to 200 kPa.

8. The method of any preceding claim, wherein a ratio between hydrogen (H₂) and carbon monoxide (CO) in the syngas produced in (S4) is adjusted by controlling the reverse Boudouard reaction depending on a flow rate of the carbon monoxide (CO) converted in (S3).

9. The method of any preceding claim, wherein a ratio between hydrogen (H₂) and carbon monoxide (CO) in the syngas produced in (S4) is adjusted by supplying separate carbon dioxide to (S3) depending on a flow rate of the carbon monoxide (CO) converted in (S3).

10. The method of any preceding claim, wherein the organic wastes in (S1) are at least one selected from waste plastic, solid wastes, biomass, waste oil, or waste tires.

11. The method of any preceding claim, further comprising: before (S2), purifying the first mixed gas produced in (S1).

12. An apparatus for manufacturing syngas, the apparatus comprising:
a first reactor (100) configured to receive organic wastes and allow for a methane reforming reaction and a gasification reaction to simultaneously occur under a catalyst;
a carbon dioxide separation unit (200) configured to receive a product from the first reactor (100) and to separate carbon dioxide therefrom;
a second reactor (300) configured to receive carbon dioxide separated in the carbon dioxide separation unit (200) and allow for a reverse Boudouard reaction to occur; and
a syngas production unit (400) configured to receive and mix a product from which the carbon dioxide has been removed in the carbon dioxide separation unit (200) and carbon monoxide converted in the second reactor (300) to produce syngas.

13. The apparatus of claim 12, wherein the first reactor (100) and/or the second reactor (300) include a fluidized bed reactor.

14. The apparatus of claim 12 or 13, further comprising:
a purification unit (500) between the first reactor (100) and a carbon dioxide storage unit; and/or
a cyclone (600) configured to separate the catalyst from the product of the first reactor (100) between the first reactor (100) and the carbon dioxide separation unit (200); and/or
a supply line configured to supply the catalyst separated from the cyclone (600) to the second reactor (300); and/or
a recirculation line configured to resupply the regenerated catalyst from the second reactor (300) to the first reactor (100).

15. A method of manufacturing a liquid hydrocarbon, the method comprising:
manufacturing syngas by a method of any one of claims 1 to 11,
supplying the syngas to a third reactor (700); and
performing a Fischer-Tropsch synthesis reaction in the third reactor.
